# EUROPEAN PATENT SPECIFICATION

(11) **EP 4 450 634 B1**
(45) Date of publication and mention of the grant of the patent: **30.09.2026**
(21) Application number: 22906632.9
(22) Date of filing: 15.12.2022
(51) Int. Cl.: C12Q 1/6806, C12Q 1/6844, C12Q 1/6869, C12N 9/22, C12Q 1/6855

(54) **METHOD FOR DETECTING INTEGRATION SITE**
VERFAHREN ZUR ERKENNUNG EINER INTEGRATIONSSTELLE
PROCÉDÉ DE DÉTECTION DE SITE D'INTÉGRATION

(30) Priority: 15.12.2021 CN 202111535111
(43) Date of publication of application: 23.10.2024
(73) Proprietor: Nanjing GenScript Biotech Co., Ltd., Nanjing, Jiangsu 211122 (CN)
(72) Inventor: HUANG, Hao, Nanjing, Jiangsu 211122 (CN); FAN, Long, Nanjing, Jiangsu 211122 (CN); MAO, Yibo, Nanjing, Jiangsu 211122 (CN); LIU, Jiadong, Nanjing, Jiangsu 211122 (CN); LI, Hong, Nanjing, Jiangsu 211122 (CN)
(74) Representative: Dantz, Jan Henning
(86) International application number: PCT/CN2022/139202
(87) International publication number: WO 2023/109887

(56) References cited:
- WO-A1-2016/082129
- WO-A1-2020/061903
- WO-A1-2021/110878
- CN-A- 101 310 024
- CN-A- 104 480 534
- CN-A- 106 191 235
- CN-A- 110 093 454
- CN-A- 112 795 620
- CN-A- 113 046 835
- ALQUEZAR-PLANAS DAVID E. ET AL: "DNA sonication inverse PCR for genome scale analysis of uncharacterized flanking sequences", vol. 12, no. 1, 18 October 2020 (2020-10-18), pages 182 - 195, XP093145320, ISSN: 2041-210X, Retrieved from the Internet <URL:https://onlinelibrary.wiley.com/doi/full-xml/10.1111/2041-210X.13497> DOI: 10.1111/2041-210X.13497
- CICERA R. LAZZAROTTO ET AL: "Defining CRISPR-Cas9 genome-wide nuclease activities with CIRCLE-seq", NATURE PROTOCOLS, vol. 13, no. 11, 19 October 2018 (2018-10-19), GB, pages 2615 - 2642, XP055556001, ISSN: 1754-2189, DOI: 10.1038/s41596-018-0055-0
- DAWES JOANNA C., WEBSTER PHILIP, IADAROLA BARBARA, GARCIA-DIAZ CLAUDIA, DORE MARIAN, BOLT BRUCE J., DEWCHAND HAMLATA, DHARMALINGAM: "LUMI-PCR: an Illumina platform ligation-mediated PCR protocol for integration site cloning, provides molecular quantitation of integration sites", MOBILE DNA, vol. 11, no. 1, 1 December 2020 (2020-12-01), XP093073219, DOI: 10.1186/s13100-020-0201-4
- MICHEL AGNES H., RIKO HATAKEYAMA, PHILIPP KIMMIG, MERET ARTER, MATTHIAS PETER, JOAO MATOS, CLAUDIO DE VIRGILIO, BENOÎT KORNMANN: "Functional mapping of yeast genomes by saturated transposition", ELIFE, vol. 6, 1 January 2017 (2017-01-01), pages e23570, XP093073220, DOI: 10.7554/eLife.23570
- JIAO, YANMEI ET AL.: "Establishment of a Method for Detection of HIV-1 Integration Sites by Inverse PCR", JOURNAL OF CAPITAL MEDICAL UNIVERSITY, vol. 30, no. 5, 31 October 2009 (2009-10-31), XP009547217
- XIE, JUN ET AL.: "Analysis of Integration Sites Flanking Sequences in hTF Transgenic Mice by Optimized Inverse PCR", CHINESE MEDICINAL BIOTECHNOLOGY, vol. 6, no. 3, 30 June 2011 (2011-06-30), XP009547218

## Description

### Cross Reference to Related Application

US 2025/340931 A1 of the same patent family is incorporated herein by reference in its entirety.

### Technical Field

The present invention relates to the technical field of molecular biology, and in particular, to a method for detecting an integration site.

### Background Art

Since its emergence in the 1980s, transgenic technology has found widespread applications, such as establishment of animal models of human diseases, *in vivo* studies of gene functions, breeding selection of genetic engineering and animal husbandry production, and is also widely used in fields such as biopharmaceuticals. Especially after the completion of the Human Genome Project and numerous model organism genome sequencing projects, the study of gene functions, namely functional genomics, has emerged as both the greatest opportunity and challenge for medical research and life science research in the 21st century. Transgenic animal technology has become one of the most important technical means for systematically researching gene function, transcriptional and expression regulations, embryonic development, etc. in living organisms, understanding the pathogenesis of human diseases, and screening new drugs or new therapies. It provides a vital technical support for the advancement of functional genomics.

At present, transgenic animal technology is primarily applied to valuable protein production, gene therapy, organ transplantation, animal variety improvement, disease modelling, etc. The successful construction of a transgenic animal model and the determination of the integration site of an exogenous gene are important prerequisites for subsequent studies of the functions and phenotypes of the exogenous gene. The determination of the integration site of the exogenous gene after CAR-T editing (CRISPR Cas9) is one of the important prerequisites for the subsequent study of the function and phenotype of the exogenous gene. Meanwhile, numerous tumour studies (such as those on human papilloma virus (HPV) and hepatitis B virus (HBV)) have confirmed that the virus can integrate into the host genome via partial sequences and the integration leads to up- or down-regulation of related gene expression and instability of the chromosome, thereby transforming normal cells into immortalized tumour cells. Moreover, many cancers are associated with the insertions of viral sequences. The study of the integration relationship between viruses and hosts (human genomes and other animal and plant genomes) has significant scientific importance and commercial value. It helps elucidate the occurrence and progression mechanisms of virus-related tumours and facilitates the study of the related conditions caused by viruses.

Viral vector-based gene transduction technology represents the most mature biological method in gene therapy. Among them, retrovirus vectors can be directly integrated into a host cell genome and can be stably expressed for a long time. Due to these features, retrovirus vector has become the core technology in clinical gene therapy. Common methods for detecting the integration site of a viral vector in a host chromosome comprise conventional PCR, Southern Blot, DNA hybridization capture and LAM-PCR (Linear-amplification mediated PCR). These methods all require a sufficient amount of sample DNAs comprising a high copy number of integrated viral vectors to acquire useful sequence information and are limited by the size, location and conformation of the target sequence, resulting in a poor sensitivity. Conventional PCR detects the integration site of an exogenous gene by amplifying a DNA fragment of the exogenous gene *in vitro.* This method requires a smaller number of samples, is simple and convenient to operate and exhibits a high sensitivity. As a result, it is the most common detection method. However, this method is likely to have problems such as false positives and low reproducibility. Southern Blot detects the presence of a DNA fragment of an exogenous gene in a sample by using an exogenous gene-specific probe, which hybridizes with a denatured DNA strand that is attached to a solid phase support and has been digested enzymatically and separated by electrophoresis. This method is highly sensitive and accurate but is quite complicated to operate and expensive. The DNA hybridization capture method detects the integration and insertion of an exogenous gene in a sample by liquid phase hybridization capture using an exogenous gene-specific probe and the DNA sample to be detected. To avoid false positives, the exogenous gene-specific probe must have no homology with the host genomic DNA. This method is simple and convenient, does not require high sample purity, and is particularly well-suited for rough screening of a large number of offspring animals. However, this method cannot accurately locate the exogenous gene. The discovery of LAM-PCR (Linear-amplification mediated PCR) represents a major advancement in identifying viral insertion sites, and the existing VIS (viral insertion site analysis) method predominantly relies on this method. LAM-PCR can be used to detect rare insertion sites in complex samples such as those from peripheral blood. However, due to the preference in the cleavage recognition frequency of restriction enzymes, this method will introduce technical errors. Therefore, the detection results cannot accurately represent the actual insertion sites within the samples, and low-frequency insertion sites in the samples may not be detected. Moreover, this method requires a substantial amount of single clone sequencing, resulting in a lengthy experimental period.

In view of this, the present invention is provided.

ALQUEZAR-PLANAS DAVID E. ET AL: "DNA sonication inverse PCR for genome scale analysis of uncharacterized flanking sequences", vol. 12, no. 1, 18 October 2020 (2020-10-18), pages 182 - 195, XP093145320, ISSN: 2041-210X, disclose a sonication-based inverse PCR high-throughput sequencing strategy to investigate uncharacterized flanking region sequences, including those flanking mobile DNA.

WO2021/110878 A1 discloses a method for detecting an integration pattern of a virus, such as human papilloma virus (HPV) in a host genome. A method is provided encompassing selective cleavage of circularized DNA fragments carrying viral DNA with an RNA-guided endonuclease and at least one guide RNA or at least one pool of guide RNAs, followed by inverse PCR, in particular inverse long-range PCR, and sequencing ("Pooled CRISPR Inverse PCR sequencing (PCIP-seq)".

CICERA R. LAZZAROTTO ET AL: "Defining CRISPR-Cas9 genome-wide nuclease activities with CIRCLE-seq", NATURE PROTOCOLS, vol. 13, no. 11, 19 October 2018 (2018-10-19), GB, pages 2615 - 2642, XP055556001, ISSN: 1754-2189, disclose a circularization for in vitro reporting of cleavage effects by sequencing (CIRCLE-seq). Highly purified gDNA circles are treated with CRISPR-Cas9 ribonucleoprotein complexes, and nuclease-linearized DNA fragments are then ligated to adapters for high-throughput sequencing.

### Summary of the Invention

An objective of the present invention is to provide a method for detecting an integration site, which comprises:
a) subjecting host DNA containing a modified nucleic acid to fragmentation to obtain linear nucleic acid fragments;
b) subjecting the linear nucleic acid fragments to cyclization to obtain a double-stranded cyclized product, and then removing uncyclized linear nucleic acid fragments;
c) performing rolling circle amplification by taking the double-stranded cyclized product as a template and using a primer specifically bound to the modified nucleic acid;
d) subjecting the amplification product to fragmentation and establishing a sequencing library;
e) subjecting the sequencing library to on-machine sequencing to obtain a sequencing result; and
f) subjecting the sequencing result and an original sequence of the host DNA to alignment to determine the integration site of the modified nucleic acid in the host DNA.

The method provided by the present invention has the following advantages:
1) The present invention enables signal amplification of a target nucleic acid by completing the amplification and enrichment of the integration site and nearby nucleic acids through RCA (rolling circle amplification) and then establishing a library, and can achieve a high sensitivity up to one copy of a nucleic acid molecule. Therefore, the present invention has great application value and potential in nucleic acid detection. Furthermore, more effective detection data are provided, with increased coverage and sequencing depth, which greatly streamlines the later-stage bioinformatics analysis process. The relative location and/or copy number information of the integration site can be efficiently and accurately obtained in conjunction with bioinformatics analysis and other means, thereby achieving a high efficiency and short period, and the acquired data are easier to store and manage.
2) The integration site region is accurately captured and enriched using a primer specifically bound to the modified nucleic acid, and with the aid of sequencing and bioinformatics analysis methods, the detection method can be used to detect all integration sites, including low-frequency integration sites, with the accuracy up to single-base resolution, and SNP (single nucleotide polymorphism) and InDel (insertion deletion marker) can also be accurately identified. This indicates that the method for detecting an integration site in the present invention achieves a high sensitivity and provides a powerful detection and application tool for the comprehensive study of integration site information in the genome.
3) Compared with those referring to whole genome re-sequencing, the method of the present invention can provide more reliable and comprehensive integration site information with the same amount of data, and achieve more accurate detection results while saving time and labour.

### Brief Description of the Drawings

To describe the technical solutions in detailed description of embodiments of the present invention or in the prior art more clearly, the accompanying drawings required in the description of detailed description of embodiments or the prior art are briefly described below. It is clear that the accompanying drawings in the following description illustrate merely some embodiments of the present invention, and those of ordinary skill in the art may further derive other drawings from these accompanying drawings without creative efforts.

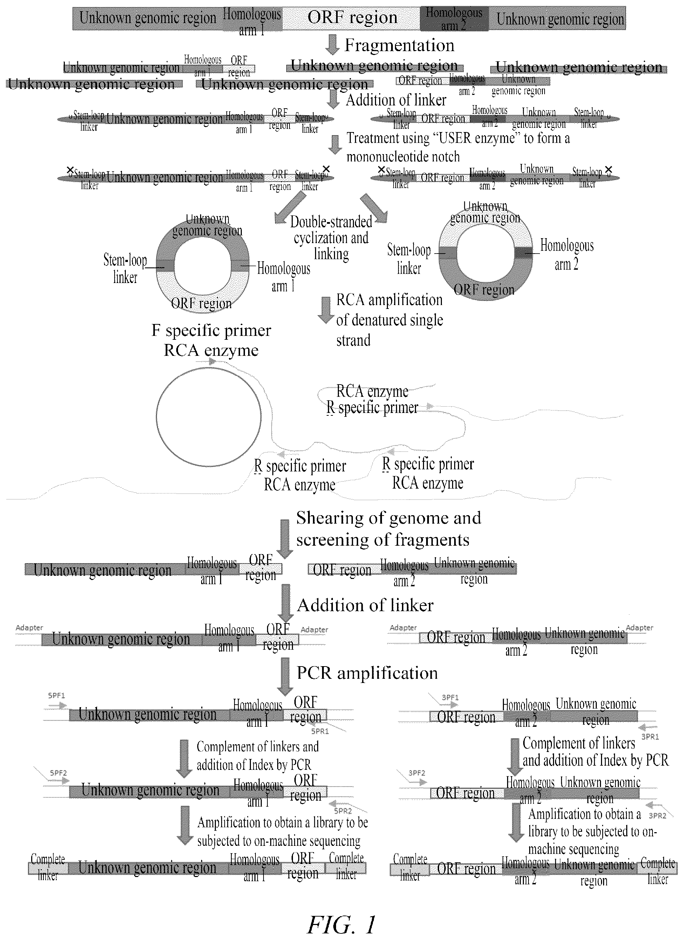

Fig. 1 is a schematic flow diagram of library construction technology;
Fig. 2 is a detection map of LTRs and AAVS1 gDNA provided in one of the Examples of the present invention; Lane M: gDNA Marker; Lane 1: LTRs gDNA; Lane 2: AAVS1 gDNA;
Fig. 3 is a map of the product peak after RCA amplification and purification of AAVS1 provided in one of the Examples of the present invention;
Fig. 4 is a map of the library peak after PCR amplification and purification of AAVS1 provided in one of the Examples of the present invention;
Fig. 5 is a map of the product peak after RCA amplification and purification of LTRs provided in one of the Examples of the present invention;
Fig. 6 is a map of the library peak after PCR amplification and purification of LTRs provided in one of the Examples of the present invention;
Fig. 7 is a map of an agarose gel electrophoresis detection of Chr19:55115264 provided in one of the Examples of the present invention; Lane M: Marker; Lane 1: target fragments;
Fig. 8 is a map of the detection peak of NW_003614611.1 (by Sanger) provided in one of the Examples of the present invention.

### Detailed Description of Embodiments

Reference will now be made in detail to the embodiments of the present invention, one or more examples of which are described below. Each example is provided by way of explanation, not limitation of the present invention. Indeed, it will be apparent to those skilled in the art that various modifications and variations can be made in the present invention without departing from the scope of the present invention. For example, features illustrated or described as part of one embodiment can be used in another embodiment to yield a still further embodiment.

Unless otherwise defined, all terms used in disclosing the invention, including technical and scientific terms, have the meaning as commonly understood by one of ordinary skill in the art to which this invention belongs. The following definitions serve to better appreciate the teachings of the present invention by way of further guidance. Herein, the terms used in the description of the present invention are merely for the purpose of describing specific examples but are not intended to limit the present invention.

The terms "and/or" and "or/and" used herein are selected to encompass any one of two or more associated items listed therein, as well as any and all combinations of the associated items listed therein, wherein the combinations include combinations of any two of the associated items listed therein, any more of the associated items listed therein, or all of the associated items listed therein. It should be noted that when at least three items are connected by a combination of at least two conjunctions selected from "and/or" and "or/and", it should be understood that in the present application, the technical solutions definitely include the technical solution in which items are all connected by "logic AND" and also definitely include the technical solutions in which items are all connected by "logic OR". For example, "A and/or B" includes the three parallel solutions of A, B and A+B. As another example, the technical solution of "A, and/or, B, and/or, C, and/or, D" includes any one of A, B, C, and D (i.e., the technical solutions in which items are all connected by "logic OR"), also includes any and all combinations of A, B, C, and D, that is, combinations of any two or any three of A, B, C, and D, and further includes the combination of all the four items A, B, C, and D (i.e., the technical solution in which items are all connected by "logic AND").

The terms "contain", "comprise", and "include" used herein are synonymous, inclusive or open-ended, and do not exclude additional, unrecited members, elements, or method steps.

Numerical ranges expressed by endpoints in the present invention include all numbers and fractions included within the range, as well as the recited endpoints.

In the present invention, expressions involving terms such as "a plurality of" and "multiple" refer to a quantity greater than or equal to 2, unless otherwise specified.

In the present invention, the technical features described in an open-ended manner include both a closed technical solution consisting of the listed features and an open-ended technical solution comprising the listed features.

In the present invention, the expressions "preferably", "preferentially", "more preferably", and "appropriately" are solely used for describing better embodiments or examples, and it should be understood that the scope of the present invention is not intended to be limited.

In the present invention, there is no intended difference in length between the terms "nucleic acid" and "oligonucleotide", both of which are N-glycosides of purine or pyrimidine bases, or modified purine or pyrimidine bases, and nucleic acids do not contain U bases, unless specifically emphasized. However, the expression "nucleic acid fragment" is a concept relative to a single nucleotide, and generally means a nucleic acid strand of a specific length. These terms refer only to the primary structure of molecules. Thus, these terms include double-stranded and single-stranded DNA, as well as double-stranded and single-stranded RNA, and double-stranded RNA-DNA hybrids.

In the present invention, the term "modified nucleic acid" refers to a nucleotide sequence comprising at least one mutation (such as insertion/deletion/substitution, or a combination thereof) when compared to an unmodified nucleic acid (original sequence). The modified nucleic acid may be generated in a targeted manner or randomly, as long as the sequence of the modified nucleic acid is known and can be distinguished from the original nucleic acid sequence and amplified using specific primers (the difference can be different insertion location and/or sequence when compared with the original sequence). It should be noted that the modified nucleic acids referred to in the present application do not exclude the modification resulting from the variations caused by endogenous mutation and/or endogenous DNA repair, which generally includes two common DNA repair pathways. One pathway is referred to as non-homologous end joining (NHEJ) pathway (Bleuyard et al. (2006) DNA Repair 5: 1-12), and another pathway is referred to as homology directed repair (HDR). Typical endogenous modified nucleic acids may result from random insertion of a transposon (a primer may be directed to the transposon) or may result from a partial chromosomal variation (such as inversion, translocation, duplication, deletion of a chromosome). The typical endogenous modified nucleic acids may also be a specific sequence formed by mutation of a plurality of adjacent points, and with this variation, the modified nucleic acid can also be distinguished from the original sequence using a specific primer.

The modified nucleic acid may also be introduced exogenously, for example by insertion/knock-out/substitution, or a combination thereof. For example, the modified nucleic acid may be an inserted exogenous nucleic acid. The term "exogenous nucleic acid" according to the present invention refers to a nucleic acid (for example, DNA, RNA) that originates outside the host DNA, is introduced from an exogenous source and is integrated into the host chromosome at a single site or at multiple sites. Two or more exogenous nucleic acids can also be inserted or introduced into the host genome. For example, two or more exogenous nucleic acids can be integrated into the host chromosome at a single site or multiple sites and still be considered as two or more exogenous nucleic acids. Moreover, the two or more exogenous nucleic acids can be distinguished and amplified using different specific primers.

In the present invention, the term "integration site" refers to the relative location of the modified nucleic acid within the host DNA. In some contexts, the term may also be used to refer to the sequence formed upon introducing the modified nucleic acid, or the nucleic acid fragments located near the site where they are joined to the host DNA. In the present invention, the copy number of the integration site is expressed as the frequency at which the modified nucleic acid is integrated at the same location in the genome, and an integration site having a copy number of not more than 3 is categorized as a low-frequency integration site.

In the present invention, a linker refers to polynucleotide fragments that are linked to the two ends (usually) of a nucleic acid. In the case of a linker capable of spontaneously forming a stem-loop structure by itself, a portion which is complementarily paired with itself to form a double strand after the formation of the stem-loop structure is referred to as a "stem", and a portion which is located between two "stems" and cannot be complementarily paired with itself to form a double strand is referred to as a "loop".

In the present invention, the term "primer" refers to an oligonucleotide, whether natural or synthetic, capable of acting as a point of initiation of RNA or DNA synthesis under conditions in which synthesis of a primer extension product complementary to a nucleic acid strand is induced, i.e., in the presence of at least four different nucleoside triphosphates and a reagent for polymerization (i.e., DNA polymerase or reverse transcriptase) in an appropriate buffer and at an appropriate temperature. The primer is preferably a single-stranded oligodeoxyribonucleotide. The appropriate length of the primer depends on the intended use of the primer, but typically ranges from 15 to 35 nucleotides, preferably 17 to 22 nucleotides, such as 18, 19, 20, or 21 nucleotides. Short primer molecules generally require lower temperatures to form sufficiently stable hybridization complexes with the template. The primer need not reflect the exact sequence of the template nucleic acid but must be sufficiently complementary to hybridize with the template. The primer may incorporate additional features that increase the specificity of binding to a target sequence (for example, a modified nucleotide such as the 3' terminally alkylated nucleotides described in EP 0866071 and EP 1201768) or allow for the detection or immobilization of the primer without altering the basic property of the primer acting as a point of initiation of RNA or DNA synthesis. For example, the primer may comprise an additional nucleic acid sequence at the 5' end, which does not hybridize to the target nucleic acid, but facilitates cloning of the amplified product and/or inhibits or prevents the formation of high molecular weight products according to the present invention. The region of the primer that is sufficiently complementary to the template to hybridize is referred to herein as the hybridization region.

The term "amplification" generally refers to any process that results in an increase in the number of copies of a molecule or collection of related molecules. When applied to a polynucleotide molecule, amplification refers to the generation of multiple copies of the polynucleotide molecule or portion of the polynucleotide molecule, typically starting from a small amount of polynucleotide (for example, viral genome), where the amplified material (amplicon, PCR amplicon) is typically detectable. Amplification of polynucleotides involves various chemical and enzymatic processes. The generation of multiple DNA copies from one or several copies of template RNA or DNA molecules during a polymerase chain reaction (reverse transcription PCR, PCR), RCA reaction, etc. is a form of amplification. Amplification is not limited to the strict replication of the starting molecule. For example, the generation of multiple cDNA molecules from a limited amount of RNA in a sample using reverse transcription PCR is a form of amplification. In addition, the generation of multiple RNA molecules from a single DNA molecule during transcription is also a form of amplification.

The present invention relates to a method for detecting an integration site, which comprises:
a) subjecting host DNA containing a modified nucleic acid to fragmentation to obtain linear nucleic acid fragments;
b) subjecting the linear nucleic acid fragments to cyclization to obtain a double-stranded cyclized product, and then removing uncyclized linear nucleic acid fragments;
c) performing rolling circle amplification by taking the double-stranded cyclized product as a template and using a primer specifically bound to the modified nucleic acid;
d) subjecting the amplification product to fragmentation and establishing a sequencing library;
e) subjecting the sequencing library to on-machine sequencing to obtain a sequencing result; and
f) subjecting the sequencing result and an original sequence of the host DNA to alignment to determine the integration site of the modified nucleic acid in the host DNA.

In some embodiments, the linear nucleic acid fragments are cyclized by adding linkers at both ends and forming two complementary sticky ends to obtain the double-stranded cyclized product.

In some embodiments, the linker is a single-stranded DNA comprising a U base site and forms a stem-loop structure by itself after annealing; after the linker forms a stem-loop structure, two complementary strands of the nucleic acid fragment and the linkers at both ends form a closed loop; the nucleic acid fragment that does not form a closed loop is removed; in some embodiments, an abasic site is formed at the U base site through enzyme cleavage, such that the site of the "loop" is adjacent to the U base and complementary sticky ends are generated at the bases near the 5' end of the linkers.

In other embodiments, the linker comprises an enzymatic cleavage site, and sticky ends are generated at both ends of the linear nucleic acid fragments through enzyme cleavage by the corresponding enzymes. For example, the linker comprises a restriction endonuclease cleavage site, and sticky ends are generated through enzyme cleavage by a restriction endonuclease.

In some embodiments, at least one nucleotide (such as 1, 2, 3, or more nucleotides) at the 5' end of the linker has a phosphorylation modification;
and/or at least one nucleotide (such as 1, 2, 3, 4, 5, or more nucleotides) at the 3' end of the linker has a thio modification.

The main role of phosphorylation modification is to enhance the linking efficiency; and the main role of thio modification is to improve the stability of the 3' end of the linear linker.

In some embodiments, the nucleotide sequence of the linker is as shown in SEQ ID NO: 1.

In some embodiments, the linker has a phosphorylation modification at the first nucleotide at the 5' end and thio modifications at the second and third nucleotides at the 3' end.

The linker may be linked using a DNA ligase following end repair and A-tailing of the nucleic acid fragments. The DNA ligase comprises any one or a combination of Hi-T4^{™} heat-resistant DNA ligase, Salt-T4^{™} salt-resistant DNA ligase, Taq high-fidelity DNA ligase, 9°N^{™} DNA ligase, *E. coli* DNA ligase, T7 DNA ligase, T3 DNA ligase, T4 DNA ligase, Circligase ssDNA ligase and thermostable 5' App DNA/RNA ligase.

In some embodiments, the abasic site at the U base is formed using an enzyme or an enzyme composition having uracil-DNA glycosylase activity and AP-endonuclease activity.

The term "enzyme having uracil-DNA glycosylase activity" refers to an enzyme that recognizes uracil present in single-stranded or double-stranded DNA and cleaves the N-glycosidic bond between the uracil base and deoxyribose, leaving an abasic site. Uracil-DNA glycosylases, abbreviated as "UDG" or "UNG" (EC 3.2.2.3), include mitochondrial UNG1, nuclear UNG2, SMUG1 (single-strand selective uracil-DNA glycosylase), TDG (TU mismatch DNA glycosylase), MBD4 (uracil-DNA glycosylase with a methyl binding region) and other prokaryotic and eukaryotic enzymes (see Krokan H. E. et al. "Uracil in DNA-occurrence, consequences and repair", Oncogene (2002) 21: 8935-9232).

In some preferred embodiments, the abasic site at the U base is formed using a mixture of uracil-DNA glycosylase (UDG) and DNA glycosylase-lyase Endo VIII, for example, a "User enzyme".

In some embodiments, the modified nucleic acid is an inserted exogenous nucleic acid.

Those skilled in the art can insert a sequence containing the modified nucleic acid into a host by any method known in the art, such as, but not limited to, transient transfection, agrobacterium transformation, transfection (based on retroviruses or lentiviruses), electroporation, microinjection, particle-mediated delivery, topical administration, delivery via cell-penetrating peptides or direct delivery mediated by mesoporous silica nanoparticles (MSNs). Optionally, the modified nucleic acid may further comprise a homologous nucleotide sequence flanking at least one nucleotide modification, wherein the flanking homologous nucleotide sequence provides sufficient homology to the nucleotide sequence to be edited.

In some embodiments, the modified nucleic acid is inserted using a gene editing method. In some embodiments, the gene editing method is based on any one of the following technologies or a combination thereof: homologous recombination, nuclease-based editing method and viral-based transfection method. In some aspects, the modified portion may occur in a non-coding region, an enhancer, a promoter, an intron, an untranslated region ("UTR") or a coding region.

The nuclease-based editing method comprises inducing editing with a nuclease (typically an endonuclease) selected from the following: nucleases, including a range of different enzymes, such as restriction endonucleases (see, for example, Roberts et al. (2003) Nucleic Acids Res 1: 418-20), Roberts et al. (2003) Nucleic Acids Res 31: 1805-12 and Belfort et al. (2002) in Mobile DNA II, pages 761-783, editors Craigie et al. (ASM Press, Washington, D.C.)), meganucleases (see, for example, WO 2009/114321; Gao et al (2010) Plant Journal 1: 176-187), TAL effector nucleases or TALENs (see, for example, US 20110145940, Christian, M., T. Cermak et al. 2010. Targeting DNA double-strand breaks with TAL effector nucleases. Genetics 186 (2): 757-61 and Boch et al. (2009), Science 326 (5959): 1509-12), zinc-finger nucleases (see, for example, Kim, Y.G., J. Cha, et al. (1996) "Hybrid restriction enzymes: zinc finger fusions to FokI cleavage ") and CRISPR-associated nucleases (see, for example, WO 2007/025097 published on March 1, 2007).

Among them, endonucleases are enzymes that cleave phosphodiester bonds within a polynucleotide chain. Endonucleases include restriction endonucleases that cleave DNA at specific sites without damaging bases; and include meganucleases, also known as homing endonucleases (HEases), which like restriction endonucleases, bind and cleave at a specific recognition site, however the recognition sites for meganucleases are typically longer, with about 18 bp or more (patent application PCT/US12/30061, filed on March 22, 2012). Based on conserved sequence motifs, meganucleases have been classified into four families, i.e., the LAGLIDADG, GIY-YIG, H-N-H, and His-Cys box families. These motifs participate in the coordination of metal ions and hydrolysis of phosphodiester bonds. HEases are notable for their long recognition sites, and also for being tolerant of some sequence polymorphisms in their DNA substrates. The naming convention for meganucleases is similar to that for other restriction endonucleases. Meganucleases are also characterized as prefixes F-, I-, or PI- for the enzymes encoded by independent ORFs, introns, and inteins, respectively. One step in the recombination process involves polynucleotide cleavage at or near the recognition site. Cleavage activity can be utilized to generate double-strand breaks. For an overview of site-specific recombinases and recognition sites thereof, see Sauer (1994) Current Opinion in Biotechnology 5: 521-7; and SadowSki (1993) FASEB 7: 760-7. In some examples, the recombinase is from the integrase or resolvase family.

Zinc-finger nucleases (ZFNs) are engineered double-strand break-inducing agents consisting of a zinc-finger DNA-binding domain and a double-strand break-inducing agent domain. Recognition site specificity is conferred by a zinc finger domain, which typically comprises two, three, or four zinc fingers, for example, having a C2H2 structure; however, other zinc finger structures are known and have been engineered. The zinc finger domain is suitable for designing polypeptides that specifically bind to the selected polynucleotide recognition sequence. ZFNs include engineered DNA-binding zinc finger domains linked to a non-specific endonuclease domain (for example, a nuclease domain from a type IIs endonuclease such as FokI). Additional functionalities may be fused to the zinc finger binding domain and include a transcriptional activator domain, a transcriptional repressor domain, and a methylase. In some examples, dimerization of nuclease domains is required for cleavage activity. Each zinc finger recognizes three consecutive base pairs in the target DNA. For example, the 3-finger domain recognizes a sequence of 9 contiguous nucleotides, and two sets of zinc finger triplets are used to bind to an 18-nucleotide recognition sequence due to the dimerization requirement of the nuclease.

The term "CRISPR-associated nucleases" herein refers to a protein or protein complex encoded by a Cas gene. The Cas endonucleases disclosed herein, when in complex with a suitable polynucleotide component, are capable of recognizing, binding to, and optionally nicking or cleaving all or part of a specific DNA target sequence. Cas endonucleases described herein comprise one or more nuclease domains. Cas endonucleases herein include those having an HNH or HNH-like nuclease domain and/or a RuvC or RuvC-like nuclease domain. The CRISPR-associated nuclease of the present invention can include Cas9 protein, Cpf1 protein, C2c1 protein, C2c2 protein, C2c3 protein, Cas3, Cas5, Cas7, Cas8, Cas10 or complexes thereof, as well as modified forms thereof.

In some embodiments, the modified nucleic acid is an inserted or substituted exogenous nucleic acid (rather than a deletion of a fragment), and "exogenous" refers to a nucleic acid that is derived from an entity that is genotypically different from the rest of the entity with which the entity is compared. The term "exogenous nucleic acid" refers to foreign nucleic acid that is found in the genome of an organism but is not naturally occurring. In some embodiments, the exogenous nucleic acid comprises an exogenous gene. In some embodiments, the product is an interfering RNA or an aptamer. The interfering RNA may be selected from, for example, siRNA or shRNA. In some embodiments, the target gene product is a polypeptide, for example, a protein that imparts some desired characteristics to the target cells, such as a fluorescent protein that allows for cell tracking, and an enzyme that provides an activity missing or altered in the target cell. The target gene comprises, for example, a gene (nucleotide sequence) encoding a protein that is defective or absent in a recipient individual or target cell; a gene that encodes a protein having a desired biological or therapeutic effect (such as antibacterial, antiviral, or antitumor/anticancer function); a nucleotide sequence encoding an RNA that inhibits or reduces the production of a deleterious or otherwise undesirable protein (for example, a nucleotide sequence encoding an RNA interfering agent as defined above); and/or a nucleotide sequence encoding an antigenic protein.

Suitable exogenous nucleic acids include, but are not limited to, nucleic acids encoding proteins used for treating the following diseases: endocrine, metabolic, hematologic, cardiovascular, neurological, musculoskeletal, urological, pulmonary and immune disorders, including, for example, cancers, inflammatory disorders, immune disorders, and chronic and infectious disorders. The cancers may be, for example, tumours arising from lesions in any of bone, bone junctions, muscle, lung, trachea, heart, spleen, arteries, veins, blood, capillaries, lymph nodes, lymphatic vessels, lymph fluid, oral cavity, pharynx, oesophagus, stomach, duodenum, small intestine, colon, rectum, anus, appendix, liver, gallbladder, pancreas, parotid gland, sublingual gland, urinary system, kidney, ureter, bladder, urethra, ovary, fallopian tube, uterus, vagina, vulva, scrotum, testis, vas deferens, penis, eye, ear, nose, tongue, skin, brain, brain stem, medulla oblongata, spinal cord, cerebrospinal fluid, nerve, thyroid, parathyroid, adrenal gland, hypophysis, pineal gland, pancreatic islets, thymus, and gonads; the immune disorders may be, for example, systemic lupus erythematosus, multiple sclerosis, type I diabetes, psoriasis, ulcerative colitis, Sjogren syndrome, scleroderma, polymyositis, rheumatoid arthritis, mixed connective tissue disease, primary biliary cirrhosis, autoimmune haemolytic anaemia, Hashimoto thyroiditis, Addison's disease, vitiligo, Graves' disease, autoimmune myasthenia gravis, ankylosing spondylitis, allergic osteoarthritis, hypersensitivity angiitis, autoimmune neutropenia, idiopathic thrombocytopenic purpura, lupus nephritis, chronic atrophic gastritis, autoimmune infertility, endometriosis, Pasture's disease, pemphigus, discoid lupus erythematosus or dense deposit disease; and the infectious disorders may be any one of a viral, bacterial, fungal and parasitic infection, or a combination thereof.

In some embodiments, the nucleic acid fragments that do not form closed loops are removed by exonuclease digestion; and/or; the non-cyclized linear nucleic acid fragments are removed by exonuclease digestion. The exonucleases used in the two steps may be the same or different.

In some embodiments, the exonuclease is selected from one or more of the following: T5 exonuclease, exonuclease VIII, exonuclease T, T7 exonuclease, RecJf exonuclease, exonuclease VII, exonuclease V, Lambda exonuclease, exonuclease III (*E. coli*) and exonuclease I (*E. coli*). Further, the exonuclease is selected from a combination of Lambda exonuclease and exonuclease I, and is used to digest linear nucleic acid fragments that do not form closed loops.

In some embodiments, the exonuclease is Lambda exonuclease and exonuclease I.

In some embodiments, in step a) and/or step d), the fragmentation is carried out using a nuclease.

In some embodiments, the nuclease comprises *Vibrio vulnificus* nuclease and one or more of T7 endonuclease, Tn5 transposase, digestive enzyme DNase I and fragmentase. In another embodiment of the present invention, a plurality of nucleases are combined in a reaction mixture where at least one nuclease is of the type capable of introducing random nicks throughout the DNA on either strand and a second nuclease is capable of counter-nicking in the immediate vicinity of the first nick, but in the opposite strand of the DNA double helix, thus causing a double-stranded DNA break. Further, a commercial Kapa frag enzyme and the reaction system thereof (Cat. NO. KK8602) are selected for DNA fragmentation treatment.

In some embodiments, the exogenous nucleic acid comprises a homologous nucleotide sequence at the 3' end and/or a homologous nucleotide sequence at the 5' end.

In some embodiments, the homologous nucleotide sequence is 50 bp to 800 bp in length, such as 60 bp, 70 bp, 80 bp, 90 bp, 100 bp, 150 bp, 200 bp, 300 bp, 400 bp, 500 bp, 600 bp, or 700 bp in length.

In some embodiments, the starting amount of the host DNA is 100 ng or more, such as 200 ng, 300 ng, 400 ng, 500 ng, 600 ng, 700 ng, 800 ng, 900 ng, 1000 ng, 1500 ng, 2000 ng, or 2500 ng, preferably 500-2500 ng.

In some embodiments, the host DNA is nuclear DNA, chloroplast DNA, mitochondrial DNA, or genomic DNA. In some embodiments, the host DNA is genomic DNA.

In some embodiments, the host DNA is from an animal, a plant, or a microorganism.

The animal may be a mammal, preferably a primate, and more preferably a rat, a rabbit, a cow, a horse, a sheep, a dog, and a human; and may also be nematodes. The animal may also be an insect, such as a fruit fly. The plant may be, for example, *Arabidopsis thaliana,* rice, wheat, maize, soybean, and cotton. The microorganism may be viruses, bacteria, and fungi (such as yeast).

Any biological sample containing host DNA can be used in the method provided by the present invention. In the present invention, the terms "biological sample", "sample", etc. refer to an animal, plant or microorganism sample; when the sample is from an animal, the sample may be a tissue or organ from an animal, or a tissue lysate; a cell (either within a subject, taken directly from a subject, or a cell maintained in culture or from a cultured cell line); a cell lysate (or lysate fraction) or cell extract; a solution containing one or more molecules derived from a cell, cellular material, or viral material (e.g. a polypeptide or nucleic acid); or a solution containing a naturally or non-naturally occurring nucleic acid, which is or can be assayed as described herein. The sample may also be any body fluid or excreta containing one or more cells, cellular components, or nucleic acids, including, but not limited to cellular, nuclear, or cell-free nucleic acids. The biological sample may also be from body fluid, including liquid, semi-solid, aerated liquid, liquid-gas mixture, etc., from an animal. Such body fluids may include, but are not limited to, saliva, sputum, serum, plasma, blood, urine, mucus, sweat, tears or other ocular fluids, otic fluids, facial fluid (such as fluid from blisters or sores), gastric fluids or gastric juices, faecal fluids, pancreatic fluids or juices, semen, products of lactation or mensuration, spinal fluids, liquid bone marrow or lymph fluid.

In some embodiments, the sequencing result and an original sequence of the host DNA are subjected to alignment to determine the integration site information of the modified nucleic acid in the host DNA and the presence or absence of a mutation in the nucleotides of the modified nucleic acid.

In some embodiments, the integration site information includes SNPs, InDels, copy number and other information of the integration site, and also includes one or more types of information involving the distribution of the integration site across different chromosomes and different regions of the genome.

In some embodiments, the mutation in the nucleotides comprises the following variations or a combination thereof: insertion of one or more nucleotides, deletion of one or more nucleotides, and substitution of one or more nucleotides.

In some embodiments, the sequencing comprises next-generation sequencing and/or first-generation sequencing.

In some embodiments, the next-generation sequencing is performed initially, followed by first-generation sequencing for validation.

The embodiments of the present invention will be described in detail below with reference to the examples. It should be understood that these examples are merely intended to illustrate the present invention but not to limit the scope of the present invention. The experimental methods in the following examples, where no specific conditions are specified, can be performed according to the instructions provided in the present invention, experimental manual or conventional conditions in the art, other experimental methods known in the art, or the conditions suggested by the manufacturer.

In the following specific examples, the measurement parameters related to the components of the raw materials may have slight deviations within the weighing accuracy, unless otherwise specified. When temperature and time parameters are involved, acceptable deviations due to instrument testing accuracy or operating accuracy are allowed.

### Example 1

The host cell used in this example was the HEK293T cell line, and all steps involving the addition of reagents during the sequencing library construction process were performed on ice.

### (1) Extraction of host cell gDNA:

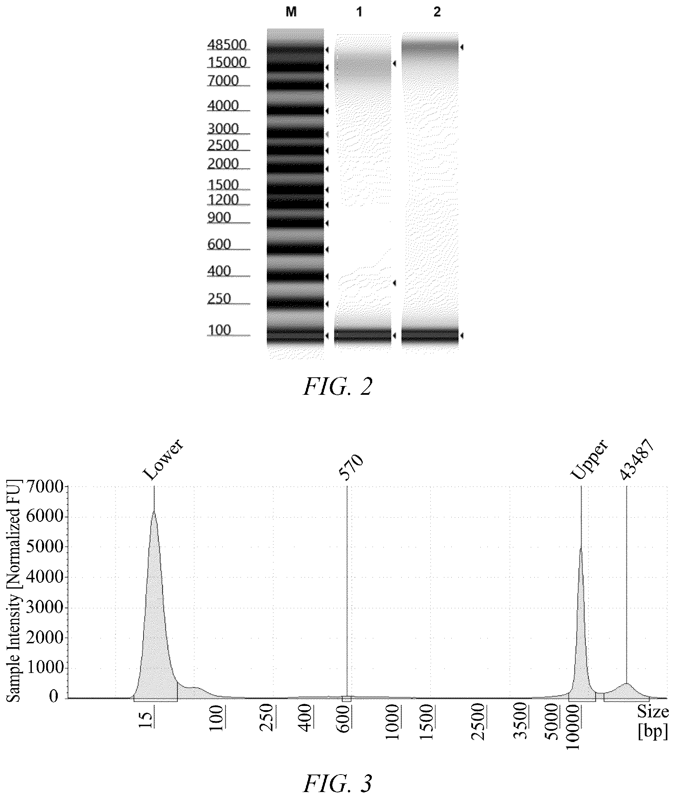

The sequence of the adeno-associated virus integration site 1 (AAVS1) was inserted into a host cell gDNA (genomic DNA). The host cell was the HEK293T cell. The host cell gDNA was extracted using MiniBEST Universal Genomic DNA Extraction Kit Ver. 5.0 (Cat. NO. 9765). The sample was designated as AAVS1 gDNA, and the concentration of AAVS1 gDNA was detected to be 93.2 ng/µL by Qubit 4.0 fluorometer. The integrity of AAVS1 gDNA was detected using the Agilent 4200 TapeStation instrument, the detection peak map is shown in Fig. 2, and it can be seen from the detection map of the 4200 instrument shown in Fig. 2 that AAVS1 gDNA exhibited good integrity, had no degradation, and can be used in subsequent experiments.

AAVS1 comprises a homologous nucleotide sequence at the 3' end and a homologous nucleotide sequence at the 5' end; and the homologous nucleotide sequence comprises 50-800 nucleotides.

The inserted AAVS1 sequence is as follows:

aagcttgggagttccgcgttacataacttacggtaaatggcccgcctggctgaccgcccaacgacccccgcccattgacgtcaa
taatgacgtatgttcccatagtaacgccaatagggactttccattgacgtcaatgggtggagtatttacggtaaactgcccacttggcagta
catcaagtgtatcatatgccaagtacgccccctattgacgtcaatgacggtaaatggcccgcctggcattatgcccagtacatgaccttat
gggactttcctacttggcagtacatctacgtattagtcatcgctattaccatggtgatgcggttttggcagtacatcaatgggcgtggatag
cggtttgactcacggggatttccaagtctccaccccattgacgtcaatgggagtttgttttggcaccaaaatcaacgggactttccaaaat
gtcgtaacaactccgccccattgacgcaaatgggcggtaggcgtgtacggtgggaggtctatataagcagagctcgtttagtgaaccgt
cagatcgcctggccgccaccatggtgagcaagggcgaggagctgttcaccggggtggtgcccatcctggtcgagctggacggcga
cgtaaacggccacaagttcagcgtgtccggcgagggcgagggcgatgccacctacggcaagctgaccctgaagttcatctgcacca
ccggcaagctgcccgtgccctggcccaccctcgtgaccaccctgacctacggcgtgcagtgcttcagccgctaccccgaccacatga
agcagcacgacttcttcaagtccgccatgcccgaaggctacgtccaggagcgcaccatcttcttcaaggacgacggcaactacaaga
cccgcgccgaggtgaagttcgagggcgacaccctggtgaaccgcatcgagctgaagggcatcgacttcaaggaggacggcaacat
cctggggcacaagctggagtacaactacaacagccacaacgtctatatcatggccgacaagcagaagaacggcatcaaggtgaactt
caagatccgccacaacatcgaggacggcagcgtgcagctcgccgaccactaccagcagaacacccccatcggcgacggccccgt
gctgctgcccgacaaccactacctgagcacccagtccgccctgagcaaagaccccaacgagaagcgcgatcacatggtcctgctgg
agttcgtgaccgccgccgggatcactctcggcatggacgagctgtacaagtaa (SEQ ID NO: 2)

Homologous nucleotide sequence at the 3' end: tcctggcagggctgtggtgaggaggggggtgtccgtgtggaaaactccct (SEQ ID NO: 3)

Homologous nucleotide sequence at the 5' end: tcctccgtgcgtcagttttacctgtgagataaggccagtagccagccccg (SEQ ID NO: 4)

### (2) Fragmentation treatment of AAVS1 gDNA:

AAVS1 gDNA was subjected to library construction, the starting amount in total for library construction was 500 ng, and Kapa frag enzyme and the reaction system thereof (Cat. NO. KK8602) were selected for fragmentation treatment to obtain fragmentation product 1. The reaction system was as follows:

| Component | Volume required for an individual reaction (µL) |
|---|---|
| KAPA Frag Buffer | 4 |
| KAPA Frag Enzyme | 8 |
| gDNA & nuclease-free water | 28 |
| Total volume | 40 |

Further, the reaction solution was loaded to a PCR instrument and the following program was run:

| Temperature | 4°C | 37°C | 75°C | 4°C |
|---|---|---|---|---|
| Time | 1 min | 20 min | 20 min | ∞ |

### (3) End repair and "A"-tailing:

After the AAVS1 gDNA fragmentation reaction was completed, the step of end repair and "A"-tailing of the library construction process was performed using the GenTrack library construction kit (Cat. NO. NGS09610) from Genscript to obtain end-repaired and "A"-tailed product 2. The reaction system was as follows:

| Component | Volume required for an individual reaction (µL) |
|---|---|
| End Prep Buffer | 7.8 |
| End Prep Enzyme | 2.2 |
| Fragmentation product 1 | 40 |
| Total volume | 50 |

Further, the reaction solution was loaded to a PCR instrument and the following program was run:

| Temperature | 37°C | 65°C | 4°C |
|---|---|---|---|
| Time | 20 min | 20 min | ∞ |

### (4) Linking of "U" (uracil) base-modified stem-loop linkers and formation of closed loop structure:

After the reaction of end repair and "A"-tailing was completed, "U" base-modified stem-loop linkers were linked to both ends of product 2 using a GenTrack library construction kit (Cat. NO. NGS09610) from Genscript, wherein the "U" base-modified stem-loop linkers were used after annealing.

The sequence of the "U" base-modified stem-loop linker is as shown below:
5'-/Phos/GTGCACGATGCGCTAG/ideoxyU/CGCATCGTGCA*C*T-3' (SEQ ID NO: 1), where "Phos" represents a phosphorylation modification, /ideoxyU/ represents uracil, and "*" represents a thio modification.

The reaction system was as follows:

| Component | Volume required for an individual reaction (µL) |
|---|---|
| Ligase Prep Buffer | 20 |
| Ligase Prep Enzyme | 4 |
| "U" base-modified stem-loop linker (40 µM) | 5 |
| Nuclease-free water | 1 |
| Product 2 | 50 |
| Total volume | 80 |

Further, the reaction solution was loaded to a PCR instrument and the following program was run (the lid temperature of the PCR instrument was set to off):

| Temperature | 23°C | 4°C |
|---|---|---|
| Time | 60 min | ∞ |

Purification step: DNA purification magnetic beads (Shanghai Yeasen Biotechnology Co., Ltd., Cat. NO.: 12601ES75) were taken out 30 min in advance, placed at room temperature, and shaken and mixed well before use; and after the linking reaction of the "U" base-modified stem-loop linker was completed, purification was carried out using 80 µL of DNA purification magnetic beads (1X), which were mixed well with the product. The mixture was left to stand for 5 min at room temperature, then the sample was placed on a magnetic stand and left to stand for 2 min, the supernatant was removed, and the beads were washed twice with 80% ethanol and eluted with 40 µL of nuclease-free water to obtain "U" base-modified stem-loop linker-containing product 3.

### (5) Digestion treatment of linear sequences without linkers:

After the purification was completed, exonuclease I and the reaction system thereof (NEB, Cat. NO. M0293L) and Lambda exonuclease (NEB, Cat. NO. M0262L) were selected to digest the nucleic acid fragments that did not form closed loops within product 3. The reaction system was as follows:

| Component | Volume required for an individual reaction (µL) |
|---|---|
| Exonuclease I reaction buffer (10X) | 5 |
| Exonuclease I (20 U/µL) | 1 |
| Lambda exonuclease (5 U/µL) | 4 |
| Product 3 | 40 |
| Total volume | 50 |

Further, the reaction system was loaded to a PCR instrument and the following program was run:

| Temperature | 37°C | 75°C | 4°C |
|---|---|---|---|
| Time | 30 min | 10 min | ∞ |

Purification step: The purification was the same as step (4). Product 4 was obtained.

### (6) Digestion treatment of "U" base by "User enzyme":

After the purification was completed, the "U" base was digested with "User enzyme" (NEB, M5505L), T4 polynucleotide kinase (T4 PNK) (NEB, M0201L) and T4 DNA ligase buffer (NEB, B0202S), and sticky ends were generated by performing cleavage at the "U" base. The reaction system was as follows:

| Component | Volume required for an individual reaction (µL) |
|---|---|
| T4 DNA ligase buffer (10X) | 5 |
| User enzyme (1 U/µL) | 3 |
| T4 Polynucleotide kinase (T4 PNK) (10 U/µL) | 2 |
| Product 4 | 40 |
| Total volume | 50 |

Further, the reaction solution was loaded to a PCR instrument and the following program was run:

| Temperature | 37°C | 4°C |
|---|---|---|
| Time | 30 min | ∞ |

Purification step: The purification was the same as step (4). Product 5 with sticky ends at both ends was obtained.

### (7) Double-stranded cyclization and linking treatment after cleavage of "U" base-modified linkers:

After the purification was completed, product 5, which had the "U" base-modified linkers cleaved and had two sticky ends, was subjected to double-stranded cyclization and linking treatment using T4 DNA ligase and the reaction system thereof (NEB, M0202L). The reaction system was as follows:

| Component | Volume required for an individual reaction (µL) |
|---|---|
| T4 DNA ligase buffer (10X) | 5 |
| PEG 4000 | 5 |
| T4 DNA ligase (30 Wiess/µL) | 4 |
| Nuclease-free water | 6 |
| Product 5 | 30 |
| Total volume | 50 |

Further, the reaction system was loaded to a PCR instrument and the following program was run (the lid temperature of the PCR instrument was set to off):

| | | |
|---|---|---|
| Temperature | 23°C | 4°C |
| Time | 60 min | ∞ |

Purification step: The purification was the same as step (4). Product 6 containing cyclized double-stranded DNAs was obtained and used as a substrate for subsequent RCA.

### (8) Digestion treatment of linear sequences not undergoing double-stranded cyclization:

After the purification was completed, exonuclease I and the reaction system thereof (NEB, Cat. NO. M0293L) and Lambda exonuclease (NEB, Cat. NO. M0262L) were selected to digest the non-cyclized linear nucleic acid fragments within product 6. The reaction system was as follows:

| Component | Volume required for an individual reaction (µL) |
|---|---|
| Exonuclease I reaction buffer (10X) | 5 |
| Exonuclease I (20 U/µL) | 1 |
| Lambda exonuclease (5 U/µL) | 4 |
| Product 6 | 40 |
| Total volume | 50 |

Further, the reaction solution was loaded to a PCR instrument and the following program was run:

| Temperature | 37°C | 75°C | 4°C |
|---|---|---|---|
| Time | 30 min | 10 min | ∞ |

Purification step: The purification was the same as step (4). Double-stranded cyclized product 7 was obtained.

### (9) RCA (rolling circle amplification) enrichment treatment using AAVS1-specific primers:

After the purification was completed, AAVS1-specific primers were designed to perform RCA (rolling circle amplification) for enrichment and library construction. The sequences of the AAVS1-specific primers 5-RCA-AAVS1-F and 5-RCA-AAVS1-R are as follows:
5-RCA-AAVS1-F: 5'-GGCTGACCGCCCAACGA-3' (SEQ ID NO: 5);
5-RCA-AAVS1-R: 5'-TATGTAACGCGGAACTCCCAAG-3' (SEQ ID NO: 6).

Firstly, denaturation treatment was performed, and the reaction system was as follows:

| Component | Volume required for an individual reaction (µL) |
|---|---|
| Amplification buffer | 1 |
| 5-RCA-AAVS1-F | 0.5 |
| 5-RCA-AAVS1-R | 0.5 |
| Product 7 | 4.8 |
| Total volume | 6.8 |

Further, the reaction system was loaded to a PCR instrument and the following program was run:

| Temperature | 95°C | 4°C |
|---|---|---|
| Time | 2 min | ∞ |

wherein the amplification buffer comprised: 500 mM Tris-HCl, 50 mM MgCl₂, 750 mM KCl, 40 mM DTT, pH 8.2, 25°C;
after double-stranded cyclized product 7 was denatured, RCA enrichment was performed with the following reaction system:

| Component | Volume required for an individual reaction (µL) |
|---|---|
| Reaction buffer | 0.7 |
| phi29 DNA polymerase (5 U/µL) | 1 |
| Nuclease-free water | 1.5 |
| Denatured product 7 | 6.8 |
| Total volume | 10 |

wherein the reaction buffer comprised: 0.2 µL of BSA (10 mg/ml) and 0.5 µL of dNTP (2.5 mM).

Further, the reaction solution was loaded to a PCR instrument and the following program was run:

| Temperature | 30°C | 65°C | 4°C |
|---|---|---|---|
| Time | 16 h | 10 min | ∞ |

After the RCA enrichment reaction was completed, the reaction product was purified with the purification method same as step (4), the concentration of the purified product (gDNA) was detected by Qubit 4.0 fluorometer, the product was detected using the Agilent 4200 TapeStation instrument and a map of the product peak was provided and is shown in Fig. 3, which indicates that the effective target product fragment was continuously enriched and amplified after the RCA of AAVS1, such that the fragment was also increased in length.

### (10) Fragmentation, end repair, "A"-tailing and linking treatment of gDNA after RCA enrichment:

The starting amount of gDNA in total for library construction after RCA enrichment and quantification was 500 ng; the GenTrack library construction kit (Cat. NO. NGS09610) from Genscript was used for library construction; steps identical to steps (2) to (4) were then carried out for fragmentation, end repair, "A"-tailing and linking treatment; and different from step (4), the linker used was a matching sequencing linker from Illumina.

The sequencing linker sequence is as shown below and was used after annealing:
Stubby Adapter-P5: 5'-ACACTCTTTCCCTACACGACGCTCTTCCGAT*C*T-3' (SEQ ID NO: 7);
Stubby Adapter-P7: 5'-/Phos/GATCGGAAGAGCACACGTCTGAACTCCAGTCAC-3' (SEQ ID NO: 8).
"Phos" represents a phosphorylation modification that can enhance the linking efficiency, "*" represents a thio modification that can improve the stability of the 3' end of the entire annealed linker, wherein the number of the thio modification can be selected from 1-5;
the product linked to the linker was purified: the purification was the same as step (4). The purified product 8 linked to the sequencing linker was obtained.

### (11) PCR amplification using AAVS1-specific amplification primer and universal amplification primer and purification treatment:

After the linker linking and purification were completed, PCR amplification premix was formulated according to the following system, and the final concentration of both the AAVS1-specific amplification primer and the universal amplification primer was 20 µM/µL.

The AAVS1-specific amplification primer and universal amplification primer sequences are as shown below:
universal amplification primer Universal-F: 5'-TACACGACGCTCTTCCGAT-3' (SEQ ID NO: 9);
AAVS1-specific amplification primer AAVS1-Special-R: 5'-ACGTGTGCTCTTCCGATCTgggccatttaccgtaagttat-3' (SEQ ID NO: 10, the specific amplification primer is underlined).

| Component | Volume required for an individual reaction µL) |
|---|---|
| Product 8 | 20 |
| KAPA HiFi HotStart ReadyMix | 25 |
| Universal-F | 2.5 |
| AAVS1-Special-R | 2.5 |
| Total volume | 50 |

PCR reaction conditions:

| Temperature | Time | Number of cycles |
|---|---|---|
| 98°C | 2 min | 1 |
| 98°C | 20 s | 8 |
| 60°C | 30 s | |
| 72°C | 30 s | |
| 72°C | 2 min | 1 |
| 4°C | Hold | 1 |

After the amplification reaction was completed, the amplification product was purified using 50 µL of purification magnetic beads (1X), and finally eluted with 20 µL of nuclease-free water to obtain amplification product 9.

After purification, library enrichment premixes were formulated according to the following system, and the final concentration of the Illumina amplification primers was 20 µM/µL. The sequences of the Illumina amplification primers are as shown below:

### P5 amplification primer sequence:

5'-AATGATACGGCGACCACCGAGATCTACAC(Index1)ACACTCTTTCCCTACAC
GACGCTCTTCCGATCT-3' (SEQ ID NO: 11);

### P7 amplification primer sequence:

5'-CAAGCAGAAGACGGCATACGAGAT(Index2)GTGACTGGAGTTCAGACGTG
TGCTCTTCCGATC-3' (SEQ ID NO: 12).

| Component | Volume required for an individual reaction (µL) |
|---|---|
| Amplification product 9 | 20 |
| KAPA HiFi HotStart ReadyMix | 25 |
| P5 amplification primer | 2.5 |
| P7 amplification primer | 2.5 |
| Total volume | 50 |

### PCR reaction conditions:

| Temperature | Time | Number of cycles |
|---|---|---|
| 98°C | 2 min | 1 |
| 98°C | 20 s | 6 |
| 60°C | 30 s | |
| 72°C | 30 s | |
| 72°C | 2 min | 1 |
| 4°C | Hold | 1 |

After library enrichment and amplification, the products were purified to obtain the final library.

### (12) Quantitative and qualitative quality inspection of the library:

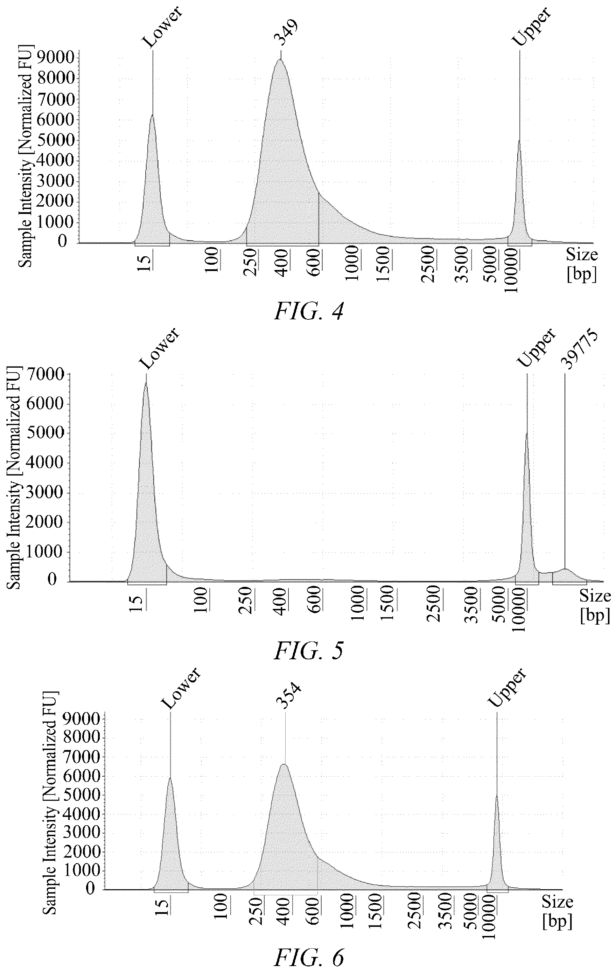

After the purification was completed, the library was quantified by Qubit 4.0 fluorometer, and qualitatively detected by the Agilent 4200 TapeStation instrument. A map of the detection peak was provided and is shown in Fig. 4, which indicates that AAVS1 met the on-machine sequencing strategy after PCR amplification and can be used for subsequent on-machine sequencing.

### (13) On-machine sequencing for the library and sequencing data analysis:

On-machine sequencing was performed for the library, the off-machine sequencing data were analysed, and the original sequencing data was subjected to quality control and filtration to obtain clean reads; the clean reads and the AAVS1 sequence (ORF region) and a reference genome were subjected to alignment and analysis; reads containing softclip alignment were extracted from the alignment result, and the corresponding softclip sequence and softclip site were obtained, wherein the softclip refers to a sequence that is not aligned to the genome but is still present in the sequencing results. Blastn search was performed on the softclip sequence within the range of the AAVS1 sequence (ORF region) and the reference genome, scoring was performed according to the results to obtain the optimal matching site, and results were output to obtain the status of the AAVS1 integration site on the genomic DNA and the specific status of the homologous nucleotide sequence, as well as the SNPs, InDels, copy number and other information of the integration site. The integration site status includes one or more types of information involving the distribution of the integration site across different chromosomes and different regions of the genome.

Upon bioinformatics analysis, AAVS1 integration sites include:
Chr19 integration site information (the Chr19 sequence is double-underlined, the wavy line-marked part indicates the homologous nucleotide sequence, and the AAVS1 sequence is single-underlined): 
CTTTGGCTCTTCACCTTTCTAGTCCCCAATTTATATTGTTCCTCCGTGCGTCAGT
TTTACCTGTGAGATAAGGCCAGTAGCCAGCCCCGAAGCTTGGGAGTTCCGCGTTAC
ATAACTTACGGTAAATGGCC (SEQ ID NO: 13)
Chr4 integration site information (the Chr4 sequence is double-underlined, the wavy line-marked part indicates the homologous nucleotide sequence, and the AAVS1 sequence is single-underlined): 
AACTTTATGAGCTGTAACACTCACCATGAAGGTCTGCAGCTTCACTCCTCCGT
GCGTCAGTTTTACCTGTGAGATAAGGCCAGTAGCCAGCCCCGAAGCTTGGGAGTT
CCGCGTTACATAACTTACGGTAAATGGCC (SEQ ID NO: 14)
Chr1 integration site information (the Chr1 sequence is double-underlined, the wavy line-marked part indicates the homologous nucleotide sequence, and the AAVS1 sequence is single-underlined): 
AAAGAACTATTTATGATATTCGTGGATCTGTAGATCAGAAATTGGGGTGGGGC
ACAGTGAGGAGGGTGTCCGTTCCTCCGTGCGTCAGTTTTACCTGTGAGATAAGGCC
AGTAGCCAGCCCCGAAGCTTGGGAGTTCCGCGTTACATAACTTACGGTAAATGGCC
(SEQ ID NO: 15)
Chr3 integration site information (the Chr3 sequence is double-underlined, the wavy line-marked part indicates the homologous nucleotide sequence, and the AAVS1 sequence is single-underlined): 
GGTTTCTCTCTCTCTCTCCATTAGCTTTTGCTGTTCACCCAAATAGACTCTCCA
GTTTATATTGTTCCTCCGTGCGTCAGTTTTACCTGTGAGATAAGGCCAGTAGCCAG
CCCCGAAGCTTGGGAGTTCCGCGTTACATAACTTACGGTAAATGGCC (SEQ ID NO:
16)
Chr6 integration site information (the Chr6 sequence is double-underlined, the wavy line-marked part indicates the homologous nucleotide sequence, and the AAVS1 sequence is single-underlined): 
GTGTTCTAAAGTGTAAACGATGAATATGGATACTTTGGCTGTTTACACAGCCTG
AATATCCTCCGTGCGTCAGTTTTACCTGTGAGATAAGGCCAGTAGCCAGCCCCGAA
GCTTGGGAGTTCCGCGTTACATAACTTACGGTAAATGGCC (SEQ ID NO: 17)
Chr2 integration site information (the Chr2 sequence is double-underlined, the wavy line-marked part indicates the homologous nucleotide sequence, and the AAVS1 sequence is single-underlined): 
AACTTTATGAGCTGTAACACTCACCATGAAGGTCTGCAGCTTCACTCCTCCGT
GCGTCAGTTTTACCTGTGAGATAAGGCCAGTAGCCAGCCCCGAAGCTTGGGAGTT
CCGCGTTACATAACTTACGGTAAATGGCC (SEQ ID NO: 18)
Chr7 integration site information (the Chr7 sequence is double-underlined, the wavy line-marked part indicates the homologous nucleotide sequence, and the AAVS1 sequence is single-underlined): 
CCCCGAGTCCCTCCTCCTGTGCTCTGAGTCCCTATTTCTGTGCCCCAATTTATAT
TGTTCCTCCGTGCGTCAGTTTTACCTGTGAGATAAGGCCAGTAGCCAGCCCCGAAG
CTTGGGAGTTCCGCGTTACATAACTTACGGTAAATGGCC (SEQ ID NO: 19)
Chr20 integration site information (the Chr20 sequence is double-underlined, the wavy line-marked part indicates the homologous nucleotide sequence, and the AAVS1 sequence is single-underlined):

CAGTGGTGGCAGTTGCCCATGCAGGGCAGCGCATGGAGACTCCAGTCAGCCA
GCCCCGTCCTCCGTGCGTCAGTTTTACCTGTGAGATAAGGCCAGTAGCCAGCCCCG
AAGCTTGGGAGTTCCGCGTTACATAACTTACGGTAAATGGCC (SEQ ID NO: 20)

It can be seen from the analysis results of the AAVS1 integration site that: the copy number of the integration site was the highest at the breakpoint Chr19:55115264; and the integration sites with low copy numbers at the chromosomal breakpoints, such as Chr1:114748679, Chr4:17765885, Chr2:21101752 and Chr3:71951874, can also be accurately captured. The results also show that the homologous nucleotide sequences at the integration sites Chr14: 90825230 and Chr5:103223897 had the two bases "TC" missed, which indicates that SNPs and Indels near the integration sites can also be accurately identified.

### (14) Validation by Agarose gel electrophoresis detection

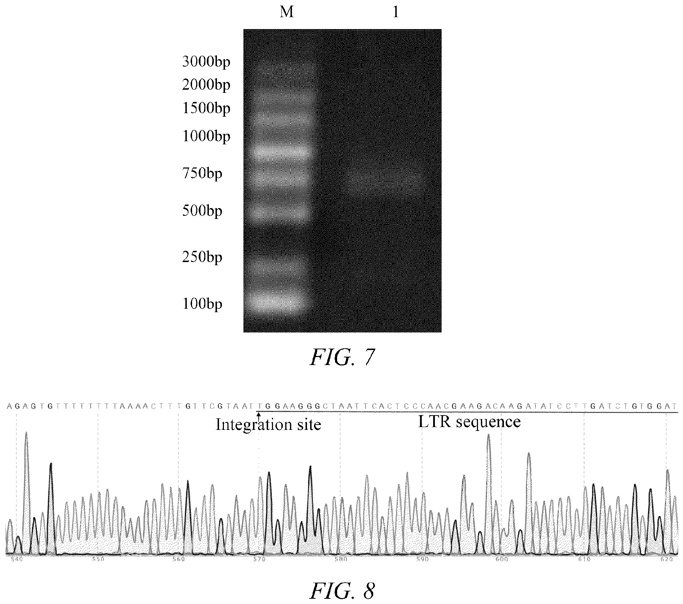

The site Chr19:55115264 was taken as an example, and agarose gel electrophoresis detection was performed for validation by designing an integration site primer. The detection gel mapis shown in Fig. 7, and it can be seen from Fig. 7 that the size of the detection fragment was consistent with that of the actual target sequence fragment.

The PCR amplification primer information is as follows:
Chr19-F: cgatggagccagagaggatc (SEQ ID NO: 21);
Chr19-R: gtcccgttgattttggtgcc (SEQ ID NO: 22).

The Chr19:55115264 (including the integration site) target fragment sequence is (the Chr19 sequence is double-underlined, the wavy line-marked part indicates the homologous nucleotide sequence, and the AAVS1 sequence is single-underlined; the target fragment is 771 bp in length):

cgatggagccagagaggatcctgggagggagagcttggcagggggtgggagggaagggggggatgcgtgacctgcccg
gttctcagtggccaccctgcgctaccctctcccagaacctgagctgctctgacgcggctgtctggtgcgtttcactgatcctggtgctgca
gcttccttacacttcccaagaggagaagcagtttggaaaaacaaaatcagaataagttggtcctgagttctaactttggctcttcacctttct
agtccccaatttatattgttcctccgtgcgtcagttttacctgtgagataaggccagtagccagccccgaagcttgggagttccgcgttaca
taacttacggtaaatggcccgcctggctgaccgcccaacgacccccgcccattgacgtcaataatgacgtatgttcccatagtaacgcc
aatagggactttccattgacgtcaatgggtggagtatttacggtaaactgcccacttggcagtacatcaagtgtatcatatgccaagtacg
ccccctattgacgtcaatgacggtaaatggcccgcctggcattatgcccagtacatgaccttatgggactttcctacttggcagtacatcta
cgtattagtcatcgctattaccatggtgatgcggttttggcagtacatcaatgggcgtggatagcggtttgactcacggggatttccaagtc
tccaccccattgacgtcaatgggagtttgttttggcaccaaaatcaacgggac (SEQ ID NO: 23)

### Example 2

The host cell used in this example was the CHO cell line, and all steps involving the addition of reagents during the sequencing library construction process were performed on ice.

Experimental steps (1)-(8) were the same as in Example 1. The host cell was the CHO cell line. The long terminal repeats (LTRs) of a lentiviral vector (HIV-1) were inserted into the host cell, and the sample was designated as LTRs gDNA. The detection peak map of LTRs gDNA from the 4200 instrument is shown in Fig. 2, and it can be seen from the detection map of the 4200 instrument shown in Fig. 2 that LTRs gDNA exhibited good integrity, had no degradation, and can be used in subsequent experiments.

The inserted LTR sequence is as follows:

tggaagggctaattcactcccaaagaagacaagatatccttgatctgtggatctaccacacacaaggctacttccctgattagca
gaactacacaccagggccaggggtcagatatccactgacctttggatggtgctacaagctagtaccagttgagccagataaggtagaa
gaggccaataaaggagagaacaccagcttgttacaccctgtgagcctgcatgggatggatgacccggagagagaagtgttagagtg
gaggtttgacagccgcctagcatttcatcacgtggcccgagagctgcatccggagtacttcaagaactgctgatatcgagcttgctacaa
gggactttccgctggggactttccagggaggcgtggcctgggcgggactggggagtggcgagccctcagatcctgcatataagcag
ctgctttttgcctgtactgggtctctctggttagaccagatctgagcctgggagctctctggctaactagggaacccactgcttaagcctca
ataaagcttgccttgagtgcttcaagtagtgtgtgcccgtctgttgtgtgactctggtaactagagatccctcagacccttttagtcagtgtg
gaaaatctctagcag (SEQ ID NO: 24)

### (9) RCA (rolling circle amplification) enrichment treatment using LTR-specific primers:

The sequences of the LTR-specific primers 5-RCA-LTRs-F and 5-RCA-LTRs-R are as follows:
5-RCA-LTRs-F: 5'-CACAAGGCTACTTCCCTGATTA-3' (SEQ ID NO: 25);
5-RCA-LTRs-R: 5'-TATCTTGTCTTCTTTGGGAGTGA-3' (SEQ ID NO: 26).

Firstly, denaturation treatment was performed, and the reaction system was as follows:

| Component | Volume required for an individual reaction (µL) |
|---|---|
| Amplification buffer | 1 |
| 5-RCA-LTRs-F | 0.5 |
| 5-RCA-LTRs-R | 0.5 |
| Double-stranded cyclized product | 4.8 |
| Total volume | 6.8 |

wherein the amplification buffer comprised: 500 mM Tris-HCl, 50 mM MgCl₂, 750 mM KCl, 40 mM DTT, pH 8.2, 25°C;

Further, the reaction solution was loaded to a PCR instrument and the following program was run.

| Temperature | 95°C | 4°C |
|---|---|---|
| Time | 2 min | ∞ |

After the double-stranded cyclized product was denatured, RCA enrichment was performed with the following reaction system:

| Component | Volume required for an individual reaction (µL) |
|---|---|
| Reaction buffer | 0.7 |
| phi29 DNA polymerase (5 U/µL) | 1 |
| Nuclease-free water | 1.5 |
| Denatured product | 6.8 |
| Total volume | 10 |

wherein the reaction buffer comprised: 0.2 µL of BSA (10 mg/ml) and 0.5 µL of dNTP (2.5 mM).

Further, the reaction solution was loaded to a PCR instrument and the following program was run.

| Temperature | 30°C | 65°C | 4°C |
|---|---|---|---|
| Time | 16 h | 10 min | ∞ |

After the RCA enrichment reaction was completed, 40 µL of nuclease-free water was added into the reaction solution; DNA Clean beads were taken out 30 min in advance, placed at room temperature, and shaken and mixed well before use; purification was carried out using 50 µL of purification magnetic beads (1X), which were mixed well with the product. The mixture was left to stand for 5 min at room temperature, then the sample was placed on a magnetic stand and left to stand for 2 min, the supernatant was removed, and the beads were washed twice with 80% ethanol and eluted with 30 µL of nuclease-free water. The concentration was detected by Qubit 4.0 fluorometer, and the product was detected using the Agilent 4200 TapeStation instrument and a map of the product peak was provided and is shown in Fig. 5, which indicates that the effective target product fragment was continuously enriched and amplified after the RCA of LTRs, such that the fragment was also increased in length.

### (10) Fragmentation, end repair, "A"-tailing and linking treatment of gDNA after RCA enrichment:

The starting amount of gDNA in total for library construction after RCA enrichment and quantification was 500 ng; the GenTrack library construction kit (Cat. NO. NGS09610) from Genscript was used for library construction; steps identical to steps (2) to (4) were then carried out for fragmentation, end repair, "A"-tailing and linking treatment; and different from step (4), the linker used was a matching sequencing linker from Illumina.

The linker sequence is as shown below and was used after annealing:
Stubby Adapter-P5: 5'-ACACTCTTTCCCTACACGACGCTCTTCCGAT*C*T-3' (SEQ ID NO: 7);
Stubby Adapter-P7: 5'-/Phos/ GATCGGAAGAGCACACGTCTGAACTCCAGTCAC-3' (SEQ ID NO: 8).
"Phos" represents a phosphorylation modification for enhancing the linking efficiency, "*" represents a thio modification that can improve the stability of the 3' end of the entire annealed linker;
after the linking of the linkers, DNA Clean beads were taken out 30 min in advance, placed at room temperature, and shaken and mixed well before use; and after the linking reaction of the linkers was completed, purification was carried out using 80 µL of purification magnetic beads (1X), which were mixed well with the product. The mixture was left to stand for 5 min at room temperature, then the sample was placed on a magnetic stand and left to stand for 2 min, the supernatant was removed, and the beads were washed twice with 80% ethanol and eluted with 20 µL of nuclease-free water.

### (11) PCR amplification using LTR-specific amplification primer and universal amplification primer and purification treatment:

After the linker linking and purification were completed, PCR amplification premix was formulated according to the following system, and the final concentration of both the LTR-specific amplification primer and the universal amplification primer was 20 µM/µL.

The LTR-specific amplification primer and universal amplification primer sequences are as shown below:
Universal amplification primer Universal-F: 5'-CACGACGCTCTTCCGAT-3' (SEQ ID NO: 27, universal amplification primer);
LTR-specific amplification primer Special-R: 5'-ACGTGTGCTCTTCCGATCTcttgtcttctttgggagtga-3' (SEQ ID NO: 28, the specific amplification primer is underlined).

| Component | Volume required for an individual reaction (µL) |
|---|---|
| Product obtained after the linker linking and purification | 20 |
| KAPA HiFi HotStart ReadyMix | 25 |
| Universal-F | 2.5 |
| Special-R | 2.5 |
| Total volume | 50 |

PCR reaction conditions:

| Temperature | Time | Number of cycles |
|---|---|---|
| 98°C | 2 min | 1 |
| 98°C | 20 s | 8 |
| 60°C | 30 s | |
| 72°C | 30 s | |
| 72°C | 2 min | 1 |
| 4°C | Hold | 1 |

After the amplification reaction was completed, the amplification product was purified using 50 µL of purification magnetic beads (1X), and finally eluted with 20 µL of nuclease-free water.

After purification, library enrichment premixes were formulated according to the following system, and the final concentration of the Illumina amplification primers was 20 µM/µL.

The sequences of the Illumina amplification primers are as shown below:
P5 amplification primer sequence: 
5'-AATGATACGGCGACCACCGAGATCTACAC(Index1)ACACTCTTTCCCTACAC
GACGCTCTTCCGATCT-3' (SEQ ID NO: 11);
P7 amplification primer sequence:

5'-CAAGCAGAAGACGGCATACGAGAT(Index2)GTGACTGGAGTTCAGACGTG
TGCTCTTCCGATC-3' (SEQ ID NO: 12);

| Component | Volume required for an individual reaction (µL) |
|---|---|
| Product obtained after the linker linking and purification | 20 |
| KAPA HiFi HotStart ReadyMix | 25 |
| P5 amplification primer | 2.5 |
| P7 amplification primer | 2.5 |
| Total volume | 50 |

PCR reaction conditions:

| Temperature | Time | Number of cycles |
|---|---|---|
| 98°C | 2 min | 1 |
| 98°C | 20 s | 6 |
| 60°C | 30 s | |
| 72°C | 30 s | |
| 72°C | 2 min | 1 |
| 4°C | Hold | 1 |

After library enrichment and amplification, DNA Clean beads were taken out 30 min in advance, placed at room temperature, and shaken and mixed well before use; and purification was carried out using 50 µL of purification magnetic beads (0.9X), which were mixed well with the product. The mixture was left to stand for 5 min at room temperature, then the sample was placed on a magnetic stand and left to stand for 2 min, the supernatant was removed, and the beads were washed twice with 80% ethanol and eluted with 30 µL of nuclease-free water.

### (12) Quantitative and qualitative quality inspection of the library:

After the purification was completed, the library was quantificationally detected by Qubit 4.0 fluorometer, and qualitatively detected by the Agilent 4200 TapeStation instrument. A map of the detection peak was provided and is shown in Fig. 6, which indicates that LTRs met the on-machine sequencing strategy after PCR amplification and can be used for subsequent on-machine sequencing. The detection peak map from Sanger for secondary validation is shown in Fig. 8.

### (13) On-machine sequencing for the library and sequencing data analysis:

On-machine sequencing was performed for the library, the off-machine sequencing data were analysed, and the original sequencing data was subjected to quality control and filtration to obtain clean reads; the clean reads and the LTR sequence (ORF region) and a reference genome were subjected to alignment and analysis; reads containing softclip alignment were extracted from the alignment result, and the corresponding softclip sequence and softclip site were obtained, Blastn search was performed on the softclip sequence within the range of the LTR sequence and the reference genome, scoring was performed according to the results to obtain the optimal matching site, and results were output to obtain the status of the LTR integration site on the genomic DNA, and the SNPs, Indels, copy number and other information of the integration site. The integration site status includes one or more types of information involving the distribution of the integration site across different nucleotide versions and different regions of the genome.

Upon bioinformatics analysis, LTR integration sites include:
NW_003613601.1 integration site information (the NW_003613601.1 sequence is double-underlined, and the wavy line-marked part indicates the LTR sequence): 
ACTTTTCTCTGGAAATGGCTTTGTGGAAGGGCTAATTCACTCCCAAAGAAGAC
AAG (SEQ ID NO: 29)
NW_003614821.1 integration site information (the NW_003614821.1 sequence is double-underlined, and the wavy line-marked part indicates the LTR sequence): 
AATGATACCCAAATCTCGGGCTAAGTTCTCCCGACCTTCAAGACCTTGAAGCT
AGAAAAAAAGATTTAGGTGTGGAAAATATAGCAGAAGCAGTCAGGTGAGAAAGA
ATTTTAGGGACTTAAAGACATCTCTAAAGTCAAAGAGGTGGAAGGGCTAATTCACT
CCCAAAGAAGACAAG (SEQ ID NO: 30)
NW_003614611.1 integration site information (the NW_003614611.1 sequence is double-underlined, and the wavy line-marked part indicates the LTR sequence): 
ATGTATACACTTAATATTCTCCAAGATTGAAGAGTGGAGATCTATTACTTACTAG
GTTTCTTATTTGTCAGCTTCATGATTTCTTCAGAGTGTTTTTTTTAAAACTTTGTTCG
TAATTGGAAGGGCTAATTCACTCCCAAAGAAGACAAG (SEQ ID NO: 31)
NW_003613734.1 integration site information (the NW_003613734.1 sequence is double-underlined, and the wavy line-marked part indicates the LTR sequence): 
TACCCTGTGCCATTCTGAAGGAAATTCAGGTTTGGAGAAGTTAGGTAATTTGT
TTCACAAGACCACACAGCTAAATGAGAAGCACTGAGATTCAAGCCCAGGACTATC
TGTGACCAGTGTTCTCCCCAGCCCTTGCCATGTCTGGAAGGGCTAATTCACTCCCA
AAGAAGACAAG (SEQ ID NO: 32)
NW_003614154.1 integration site information (the NW_003614154.1 sequence is double-underlined, and the wavy line-marked part indicates the LTR sequence): 
CTCATCTTATTTGTTAATTCAATTTTGAAATTGTTTAAACTCATACACCAAACAT
ACACATATTGTTTGTAATTCTTACCATTCTTAGACTTGTCAGCAAATCACTGTTCTGT
GATACTGGAAGGGCTAATTCACTCCCAAAGAAGACAAG (SEQ ID NO: 33)
NW_003613767.1 integration site information (the NW_003613767.1 sequence is double-underlined, and the wavy line-marked part indicates the LTR sequence): 
TATTTGTATAGTATATACGAAGAGTAGAAGACTATGGTGTTATTTTGCCCCTTAT
CTCTAAAGGCTTATTCTTGAGATATCAACACGTGGGTCTATGTATATTGATACAGATA
TTATGGAATGTTTGGAAGGGCTAATTCACTCCCAAAGAAGACAAG (SEQ ID NO: 34)
NW_003613592.1 integration site information (the NW_003613592.1 sequence is double-underlined, and the wavy line-marked part indicates the LTR sequence): 
ATTTCTATTACCAATTAATTCCTTATTAATGATTCTTGGAGTTTCAATCTGCTCTT
TAACTATTCACTATCGTAGCTCCAGGTAGTAAAACTAAAAACATTTCTTGCTAATTC
CAATGAACGTGTCTTTCTCTGGAAATGGCTTTGTGGAAGGGCTAATTCACTCCCAA
AGAAGACAAG (SEQ ID NO: 35)
NW_003615763.1 integration site information (the NW_003615763.1 sequence is double-underlined, and the wavy line-marked part indicates the LTR sequence): 
GTGTGTGTGTGTGTGTGTGTGTGTGTGGACATGTGGATGCAGGTACATGTG
TGTGTATATGGAGAGGGCAGATGTCAATCTCTGGTTTCTTCCACAGGCACCATCTAC
TCTATCTTTTGAGATAGTGGAAGGGCTAATTCACTCCCAAAGAAGACAAG (SEQ ID
NO: 36)
NW_003613979.1 integration site information (the NW_003613979.1 sequence is double-underlined, and the wavy line-marked part indicates the LTR sequence):

GAGATATAACAGCAGATGTGACAGTCATAATAAGAAAGTGTACAGGCTACTTC
TTTAAATTACAAGAAATGAGAAGCAAGAACAGTTAACATTTCCAAAATGGGACTTT

TTATAGCTGTGGTGTCCTTTAGTGGAAGGGCTAATTCACTCCCAAAGAAGACAAG
(SEQ ID NO: 37)

It can be seen from the analysis results of the LTR integration site that: the copy number of the LTR integration site was the highest at the breakpoint of version NW_003613592.1:3832341, and the integration sites at other breakpoints such as version NW_003614611.1:240752, NW_003615763.1:70718, NW_003613979.1:953965, NW_003613734.1:1543867, NW_003613767.1:128753 and NW_003614154.1:1050586 can also be accurately captured; and the integration site with low copy numbers, such as integration site NW_003613601.1: 103659, can also be accurately captured.

### (14) Validation by Sanger sequencing

The site of version NW_003614611.1:240752 was taken as an example. By designing integration site primers (SEQ ID NO: 39 and SEQ ID NO: 40), Sanger sequencing was performed for secondary validation to confirm the accuracy of the NGS results; the detection peak map from Sanger is shown in Fig. 8, and it can be concluded from Fig. 8 that: GTTCGTAATtggaaggg (SEQ ID NO:41) (the wavy line-marked part indicates part of the LTR sequence (ORF region)) is the integration site information, demonstrating the accuracy of the NGS results. The target fragment from Sanger sequencing was identical to the actual target sequence fragment, and the integration site was accurately identified.

The NW_003614611.1 sequence was validated (the NW_003614611.1 sequence is double-underlined, and the wavy line-marked part indicates the LTR sequence), and the sequence is provided as follows: 
AGTATGATAGTGTGTGTGTGTGTGTGTGTGTGTGTGTGTGTGTGTGTGTGCATG
TGTGTGTTTGTCCTCAATCTATGTACATGCTTATGTGTGCTAGTGTTTATGTATGATA
ATTTCAATTTCCTACAGTTTCTTGAATGTCAGGCAACAGCCATGGATCTTGGTTTTC
CTATTCTGCCTTGACAGAGGCAGTGTCCCTTACTGTTTATTGCTTACACAGTAAAAT
CCTGTGCCCTGTGAACCCTGTTTTCAGCCACCTTATATCATTCTTAAATGCTTCATTA
TCTGAATTTTTTATTTCTCAAAGTATCAACATGATTGAACTTCTCAACTAGGGCATTA
ACATGCAAGAAAAGACAAAATACCTGTGGCTTGCAGAGGTTCATCTTTCCAATGTA
ATTGATATCCATATAACATCTCATTTATAAATCAACCATGCTCACTAGGAAATAATAA
TCTTAATTTTAATTAATTTATTTTTATTTAAATTAGATTATTCTCTCAAACAATAGAAA
AAGATTTTTTAAATGCTTCAGGTTTGTGTTTGTTTAATTACTCTTTAGAATAAATTTT
TCTACAGAAAGTACTCAATTTTTTATTTTGAGCTAAAAACTTTCAGATAAGTCAAGG
TCTTGAGTTTTCTCTATTAGTACAAAACAGGGGGATTAGAGTTTAATGTATACACTT
 
AATATTCTCCAAGATTGAAGAGTGGAGATCTATTACTTACTAGGTTTCTTATTTGTCA
GCTTCATGATTTCTTCAGAGTGTTTTTTTTAAAACTTTGTTCGTAATtggaagggctaattcac
tcccaaagaagacaagatatccttgatctgtggatctaccacacacaaggctacttccctgattagcagaactacacaccagggccagg
ggtcagatatccactgacctttggatggtgctacaagctagtaccagttgagccagataaggtagaagaggccaataaaggagagaac
accagc (SEQ ID NO: 38)
NW_003614611.1-F primer: TCCCTTACTGTTTATTGCTT (SEQ ID NO: 39);
NW_003614611.1-R primer: GCTGGTGTTCTCTCCTTTA (SEQ ID NO: 40).

The above examples merely represent several embodiments of the present invention for the purpose of specific and detailed description, but should not be construed as limiting the scope of the present invention.

## Claims

1. A method for detecting an integration site, **characterised in that** the method comprises:
a) subjecting host DNA containing a modified nucleic acid to fragmentation to obtain linear nucleic acid fragments;
b) subjecting the linear nucleic acid fragments to cyclization to obtain a double-stranded cyclized product, and then removing uncyclized linear nucleic acid fragments;
c) performing rolling circle amplification by taking the double-stranded cyclized product as a template and using a primer specifically bound to the modified nucleic acid;
d) subjecting the amplification product to fragmentation and establishing a sequencing library;
e) subjecting the sequencing library to on-machine sequencing to obtain a sequencing result; and
f) subjecting the sequencing result and an original sequence of the host DNA to alignment to determine the integration site of the modified nucleic acid in the host DNA.

2. The method according to claim 1, **characterised in that** in step b), the linear nucleic acid fragments are cyclized by adding linkers at both ends and forming two complementary sticky ends to obtain the double-stranded cyclized product.

3. The method according to claim 2, **characterised in that** the linker is a single-stranded DNA comprising a U base site and forms a stem-loop structure after annealing, and the complementary sticky ends are generated by forming an abasic site at the U base site through enzyme cleavage.

4. The method according to claim 3, **characterised in that** the abasic site at the U base is formed using an enzyme or an enzyme composition having uracil-DNA glycosylase activity and AP-endonuclease activity; preferably a mixture (User enzyme) of uracil-DNA glycosylase and Endo VIII.

5. The method according to any one of claims 2 to 4, **characterised in that** at least one nucleotide at the 5' end of the linker has a phosphorylation modification;
and/or 1, 2, 3, 4, 5 or more nucleotides at the 3' end of the linker have a thio modification.

6. The method according to claim 5, **characterised in that** the nucleotide sequence of the linker is as shown in SEQ ID NO: 1.

7. The method according to claim 1, **characterised in that** the modified nucleic acid is an inserted exogenous nucleic acid, preferably inserted using a gene editing method.

8. The method according to claim 7, **characterised in that** the gene editing method is based on any one of the following technologies or a combination thereof:
homologous recombination, nuclease-based editing method and viral-based transfection method.

9. The method according to claim 1, **characterised in that** in step b), the uncyclized linear nucleic acid fragments are removed by exonuclease digestion.

10. The method according to claim 9, **characterised in that** in step b), the exonuclease used is selected from one or more of the following: T5 exonuclease, exonuclease VIII, exonuclease T, T7 exonuclease, RecJf exonuclease, exonuclease VII, exonuclease V, Lambda exonuclease, exonuclease III and exonuclease I; preferably Lambda exonuclease and exonuclease I.

11. The method according to claim 1, **characterised in that** in step a) and/or step d), the fragmentation is carried out using a nuclease.

12. The method according to claim 11, **characterised in that** the nuclease comprises one or more of *Vibrio vulnificus* nuclease, T7 endonuclease, Tn5 transposase, digestive enzyme DNase I and fragmentase.

13. The method according to claim 1, **characterised in that** the exogenous nucleic acid comprises a homologous nucleotide sequence at the 3' end and/or a homologous nucleotide sequence at the 5' end; and the homologous nucleotide sequence is 50 bp to 800 bp in length.

14. The method according to claim 1, **characterised in that** the starting amount of the host DNA is 100 ng or more, preferably 500 ng to 2500 ng.

15. The method according to any one of claims 1 to 14, **characterised in that** the host DNA is nuclear DNA, chloroplast DNA, mitochondrial DNA, or genomic DNA; preferably genomic DNA.

16. The method according to any one of claims 1 to 14, **characterised in that** the host DNA is from an animal, a plant, or a microorganism.

## Patentansprüche

1. Verfahren zum Nachweisen einer Integrationsstelle, **dadurch gekennzeichnet, dass** das Verfahren umfasst:
a) Aussetzen einer Wirts-DNA, die eine modifizierte Nukleinsäure enthält, einer Fragmentierung, um lineare Nukleinsäurefragmente zu erhalten;
b) Aussetzen der linearen Nukleinsäurefragmente einer Zyklisierung, um ein doppelsträngiges, zyklisiertes Produkt zu erhalten und dann Entfernen von nicht zyklisierten, linearen Nukleinsäurefragmenten;
c) Ausführen einer Rolling-Circle-Amplifikation durch Annehmen des doppelsträngigen, zyklisierten Produkts als Matrize und Einsetzen eines Primers, der spezifisch an die modifizierte Nukleinsäure gebunden ist;
d) Aussetzen des Amplifikationsprodukts einer Fragmentierung und Erstellen einer Sequenzierungsbibliothek;
e) Aussetzen der Sequenzierungsbibliothek einer geräteintegrierten Sequenzierung, um ein Sequenzierungsergebnis zu erhalten; und
f) Aussetzen des Sequenzierungsergebnisses und einer Originalsequenz der Wirts-DNA einem Alignment, um die Integrationsstelle der modifizierten Nukleinsäure in der Wirts-DNA zu bestimmen.

2. Verfahren nach Anspruch 1, **dadurch gekennzeichnet, dass** in Schritt b) die linearen Nukleinsäurefragmente zyklisiert werden durch Hinzufügen von Linkern an beiden Enden und durch Ausbilden von zwei komplementären kohäsiven Enden, um das doppelsträngige, zyklisierte Produkt zu erhalten.

3. Verfahren nach Anspruch 2, **dadurch gekennzeichnet, dass** der Linker eine einzelsträngige DNA ist, umfassend eine U-Basen-Stelle, und dass er eine nach dem Hybridisieren eine Haarnadelstruktur ausbildet, und dass die komplementären kohäsiven Enden durch Ausbilden einer abasichen Stelle an der U-Basen-Stelle mithilfe von Enzymspaltung erzeugt werden.

4. Verfahren nach Anspruch 3, **dadurch gekennzeichnet, dass** die abasische Stelle an der U-Base ausgebildet wird unter Einsatz eines Enzyms oder einer Enzymzusammensetzung mit Uracil-DNA-Glykosylaseaktivität und AP-Endonukleaseaktivität, vorzugsweise eines Gemischs (USER-Enzym) aus Uracil-DNA-Glykosylase und Endo VIII.

5. Verfahren nach einem der Ansprüche 2 bis 4, **dadurch gekennzeichnet, dass** mindestens ein Nukleotid am 5'-Ende des Linkers eine Phosphorylierungsmodifikation aufweist und/oder 1, 2, 3, 4, 5 oder mehr Nukleotide am 3'-Ende des Linkers eine Thio-Modifikation aufweisen.

6. Verfahren nach Anspruch 5, **dadurch gekennzeichnet, dass** die Nukleotidsequenz des Linkers wie in SEQ ID NO: 1 gezeigt ausgeführt ist.

7. Verfahren nach Anspruch 1, **dadurch gekennzeichnet, dass** die modifizierte Nukleinsäure eine insertierte, exogene Nukleinsäure ist, vorzugsweise mithilfe eines Gen-Editing-Verfahrens insertiert.

8. Verfahren nach Anspruch 7, **dadurch gekennzeichnet, dass** das Gen-Editing-Verfahren auf einer der folgenden Technologien oder auf einer Kombination daraus basiert: homologe Rekombination, nukleasevermitteltes Editing-Verfahren und virusbasiertes Transfektionsverfahren.

9. Verfahren nach Anspruch 1, **dadurch gekennzeichnet, dass** in Schritt b) die nicht zyklisierten, linearen Nukleinsäurefragmente mithilfe von Exonuklease-Verdau entfernt werden.

10. Verfahren nach Anspruch 9, **dadurch gekennzeichnet, dass** die in Schritt b) verwendete Exonuklease ausgewählt ist aus einem oder mehreren der Folgenden: T5-Exonuklease, Exonuklease VIII, Exonuklease T, T7-Exonuklease, RecJf-Exonuklease, Exonuklease VII, Exonuklease V, Lambda-Exonuklease, Exonuklease III und Exonuklease I; vorzugsweise Lambda-Exonuklease und Exonuklease I.

11. Verfahren nach Anspruch 1, **dadurch gekennzeichnet, dass** in Schritt a) und/oder in Schritt d) die Fragmentierung unter Einsatz einer Nuklease durchgeführt wird.

12. Verfahren nach Anspruch 11, **dadurch gekennzeichnet, dass** die Nuklease eines oder mehrere aus *Vibrio-vulnificus*-Nuklease, T7-Endonuklease, Tn5-Transposase, Verdauungsenzym DNase I und Fragmentase umfasst.

13. Verfahren nach Anspruch 1, **dadurch gekennzeichnet, dass** die exogene Nukleinsäure eine homologe Nukleotidsequenz am 3'-Ende und/oder eine homologe Nukleotidsequenz am 5'-Ende umfasst; und dass die homologe Nukleotidsequenz 50 bp bis 800 bp lang ist.

14. Verfahren nach Anspruch 1, **dadurch gekennzeichnet, dass** die Ausgangsmenge der Wirts-DNA mindestens 100 ng beträgt, vorzugsweise 500 ng bis 2500 ng.

15. Verfahren nach einem der Ansprüche 1 bis 14, **dadurch gekennzeichnet, dass** die Wirts-DNA nukleäre DNA, Chloroplasten-DNA, mitochondriale DNA oder genomische DNA ist; vorzugsweise genomische DNA.

16. Verfahren nach einem der Ansprüche 1 bis 14, **dadurch gekennzeichnet, dass** die Wirts-DNA von einem Tier, einer Pflanze oder einem Mikroorganismus stammt.

## Revendications

1. Méthode de détection d'un site d'intégration, **caractérisée en ce que** la méthode comprend de :
a) soumettre un ADN hôte contenant un acide nucléique modifié à fragmentation pour obtenir des fragments d'acides nucléiques linéaires ;
b) soumettre les fragments d'acides nucléiques linéaires à cyclisation pour obtenir un produit cyclisé à double brin, puis éliminer les fragments d'acides nucléiques linéaires non cyclisés ;
c) effectuer une amplification circulaire en prenant le produit cyclisé à double brin comme matrice et en utilisant une amorce liée spécifiquement à l'acide nucléique modifié ;
d) soumettre le produit d'amplification à fragmentation et établir une bibliothèque de séquençage ;
e) soumettre la bibliothèque de séquençage à un séquençage on-machine pour obtenir un résultat de séquençage ; et
f) soumettre le résultat de séquençage et une séquence originale de l'ADN hôte à alignement pour déterminer le site d'intégration de l'acide nucléique modifié dans l'ADN hôte.

2. Méthode selon la revendication 1, **caractérisée en ce qu'**à l'étape b), les fragments d'acide nucléique linéaires sont cyclisés par ajout de linkers aux deux extrémités et formation de deux extrémités cohésives complémentaires pour obtenir le produit cyclisé à double brin.

3. Méthode selon la revendication 2, **caractérisée en ce que** le linker est un ADN simple brin comprenant un site de base U et forme une structure tige-boucle après hybridation, et les extrémités cohésives complémentaires sont générées par formation d'un site abasique au niveau du site de base U par clivage enzymatique.

4. Méthode selon la revendication 3, **caractérisée en ce que** le site abasique au niveau de la base U est formé en utilisant un enzyme ou une composition enzymatique ayant une activité d'uracile-ADN glycosylase et une activité d'AP-endonucléase ; de préférence un mélange (enzyme User) d'uracile-ADN glycosylase et d'Endonucléase VIII.

5. Méthode selon l'une quelconque des revendications 2 à 4, **caractérisée en ce qu'**au moins un nucléotide à l'extrémité 5' du linker a une modification par phosphorylation ; et/ou 1, 2, 3, 4, 5 ou plus de nucléotides à l'extrémité 3' du linker ont une thio-modification.

6. Méthode selon la revendication 5, **caractérisée en ce que** la séquence nucléotidique du linker est comme représenté à la SEQ ID NO: 1.

7. Méthode selon la revendication 1, **caractérisée en ce que** l'acide nucléique modifié est un acide nucléique exogène inséré, inséré de préférence en utilisant une méthode d'édition génomique.

8. Méthode selon la revendication 7, **caractérisée en ce que** la méthode d'édition génomique est basée sur l'une quelconque des technologies suivantes ou une combinaison de celles-ci : recombinaison homologue, édition à partir d'une nucléase et transfection virale.

9. Méthode selon la revendication 1, **caractérisée en ce qu'**à l'étape b), les fragments d'acide nucléique linéaires non cyclisés sont éliminés par digestion par exonucléase.

10. Méthode selon la revendication 9, **caractérisée en ce qu'**à l'étape b), l'exonucléase utilisée est choisie parmi au moins une des suivantes : exonucléase T5, exonucléase VIII, exonucléase T, exonucléase T7, exonucléase RecJf, exonucléase VII, exonucléase V, exonucléase Lambda, exonucléase III et exonucléase I ; de préférence exonucléase Lambda et exonucléase I.

11. Méthode selon la revendication 1, **caractérisée en ce qu'**à l'étape a) et/ou à l'étape d), la fragmentation est effectuée en utilisant une nucléase.

12. Méthode selon la revendication 11, **caractérisée en ce que** la nucléase comprend au moins une parmi la nucléase de *Vibrio vulnificus*, l'endonucléase T7, la transposase Tn5, l'enzyme digestive DNase I et la fragmentase.

13. Méthode selon la revendication 1, **caractérisée en ce que** l'acide nucléique exogène comprend une séquence nucléotidique homologue à son extrémité 3' et/ou une séquence nucléotidique homologue à son extrémité 5' ; et la séquence nucléotidique homologue a une longueur de 50 bp à 800 bp.

14. Méthode selon la revendication 1, **caractérisée en ce que** la quantité initiale de l'ADN hôte est d'au moins 100 ng, de préférence de 500 ng à 2500 ng.

15. Méthode selon l'une quelconque des revendications 1 à 14, **caractérisée en ce que** l'ADN hôte est un ADN nucléaire, un ADN de chloroplaste, un ADN mitochondrial ou un ADN génomique ; de préférence un ADN génomique.

16. Méthode selon l'une quelconque des revendications 1 à 14, **caractérisée en ce que** l'ADN hôte provient d'un animal, d'une plante ou d'un microorganisme.
